Europäisches Patentamt

European Patent Office

Office européen des brevets

(19)

(11) Veröffentlichungsnummer: **0 027 208**
**B1**

## (12) EUROPÄISCHE PATENTSCHRIFT

(45) Veröffentlichungstag der Patentschrift:
02.06.82

(51) Int. Cl.³: **C 07 C 102/04**

(21) Anmeldenummer: **80105932.0**

(22) Anmeldetag: **01.10.80**

(54) Verfahren zur kontinuierlichen Herstellung von Di-acetyl-ethylendiamin.

(30) Priorität: **10.10.79 DE 2941023**

(43) Veröffentlichungstag der Anmeldung:
**22.04.81 Patentblatt 81/16**

(45) Bekanntmachung des Hinweises auf die Patenterteilung:
**02.06.82 Patentblatt 82/22**

(84) Benannte Vertragsstaaten:
**AT BE CH DE FR GB IT LI NL SE**

(56) Entgegenhaltungen:
**DE-A-2 118 282**
**DE-A-2 828 765**

(73) Patentinhaber: **HOECHST AKTIENGESELLSCHAFT,**
**Postfach 80 03 20, D-6230 Frankfurt/Main 80 (DE)**

(72) Erfinder: **Wellbrock, Werner, Dr., Drei Linden Strasse 30,**
**D-6232 Bad Soden am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur kontinuierlichen Herstellung von Di-acetylethylendiamin

Di-acetylethylendiamin (DAED) wird normalerweise hergestellt als Vorstufe für die Weiteracetylierung zum Tetraacetylethylendiamin (genannt TAED), welches besondere Bedeutung als Bleichaktivator in verschiedenen Niedrigtemperatur-Waschmitteln hat. Ein quasi-kontinuierliches Kaskaden-Verfahren zur Herstellung von Di-acetylethylendiamin aus Essigsäure und Ethylendiamin ist beschrieben in der Offenlegungsschrift 2 118 282. Dieses Verfahren verläuft, soweit es in dem Beispiel beschrieben ist, mindestens 2stufig. Für die Durchführung des auf Seite 2 beschriebenen Kolonnenverfahrens ist ein Überschuß an Essigsäure erforderlich. Beide Varianten sind in der Raum-Zeit-Ausbeute gebunden an die physikalisch-technischen Voraussetzungen, die ihrerseits bestimmend für die Geschwindigkeit der destillativen Entfernung des entstandenen Reaktionswassers aus der Reaktionszone sind. Auch ist es bei diesem Verfahren schwierig, das Reaktionswasser quantitativ zu entfernen, wie es für eine hohe Ausbeute bei der Weiterverarbeitung zum TAED erforderlich ist. Der noch verbleibende Restwassergehalt, der bei der Weiterverarbeitung zum TAED zur Zerstörung von Essigsäureanhydrid führt, wird mit ca. 1% angegeben.

Es wurde nun ein neues Verfahren zur vollkontinuierlichen Herstellung von Di-acetylethylendiamin gefunden, das darin besteht, daß man ein Gemisch aus Ethylendiamin und Essigsäure zunächst in einer Niedrigtemperaturzone bei ca. 80 bis 140°C vorreagieren läßt, das Reaktionsgemisch in einer nachfolgenden Hochtemperaturzone bei ca. 140 bis 215°C, vorzugsweise 160 bis 180°C, ausreagieren läßt, das gebildete Di-acetylethylendiamin am Ende der Hochtemperaturzone kontinuierlich austrägt, das entstandene Reaktionswasser unmittelbar während es entsteht im Gegenstrom mit einem Inertgas zwischen der Niedrigtemperaturzone und der Hochtemperaturzone in eine Fraktionierkolonne herausführt und vorzugsweise das Reaktionswasser fraktioniert destilliert, und die ausfraktionierten Reaktionskomponenten wieder in die Hochtemperaturzone zurückführt.

Der Vorteil gegenüber der in der Offenlegungsschrift 2 118 282 beschriebenen Verfahrensweise ist der, daß hier das entstehende Reaktionswasser als Wasserdampf nicht im Gegenstorm die eintretenden Ausgangsstoffe Essigsäure und Ethylendiamin passieren muß, da bereits in der Niedrigtemperaturzone eine Vorreaktion stattgefunden hat. Nur auf diese Art und Weise wird das Ausstrippen des Reaktionswassers mittels eines Inertgases möglich, so daß das austretende, mit Wasserdampf beladene Inertgas sich nicht im Gegenstrom mit den Ausgangsverbindungen belädt. Dies führt letztlich dazu, daß das in der geschilderten Weise hergestellte DAED praktisch quantitativ aus den Ausgangsverbindungen entsteht und zudem mit einem Restwassergehalt anfällt, der kleiner als 0,1% ist, also mindestens 10mal so niedrig wie es in der genannten Offenlegungsschrift angegeben ist.

Eine zur Durchführung des Verfahrens geeignete Apparatur ist in der beigefügten schematischen Zeichnung dargestellt. Sie besteht im wesentlichen aus den beiden Füllkörperkolonnen (1) und (3), die heizbar bzw. kühlbar sind, und den beiden anschließenden Heizzonen (2) bzw. (4) sowie der Fraktionierkolonne (5). In die erste Füllkörperkolonne (1), die als Vorreaktor dient und auf einer Temperatur von ca. 80 bis 140°C, vorzugsweise ca. 90°C, gehalten wird und als Niedrigtemperaturzone bezeichnet wird, wird über die Leitung (6) ein Gemisch aus Essigsäure und Ethylendiamin eingegeben. Das Verhältnis Essigsäure zu Ethylendiamin beträgt 2 : 1. Ein Überschuß an Essigsäure ist unschädlich aber auch unnötig. Beide Ausgangskomponenten können in wasserfreier Form oder aber auch als wäßrige Lösungen eingesetzt werden.

Im Anschluß an diese erste Füllkörperkolonne (1) durchläuft das Reaktionsgemisch eine Heizzone (2), wo das Reaktionsgemisch auf eine Temperatur von ca. 140 bis 215°C aufgeheizt wird. Das Reaktionsgemisch geht dann in eine zweite Füllkörperkolonne (3) mit nachgeschalteter Heizzone (4), wo man das Reaktionsgemisch auf der angegebenen Temperatur von 140 bis 215°C hält und ausreagieren läßt. An diese letzte Heizzone können sich gegebenenfalls noch weitere Einheiten aus Füllkörperkolonne und Heizzone vom gleichen Typ wie zuvor anschließen. Am Ende der letzten Heizzone wird das entstandene Di-acetylethylendiamin über die Leitung (7) kontinuierlich ausgetragen. Die Heizzonen sind vorzugsweise als Zonenheizung mit Zwangsumlauf ausgestattet, wo das Reaktionsgemisch an der äußeren und inneren Fläche der Heizung vorbeigeführt wird.

In die Temperaturzone von 140 bis 215°C — hier mit Hochtemperaturzone bezeichnet — wird über Leitung (8) ein Inertgas, vorzugsweise Stickstoff, eingeführt. Mit Hilfe dieses Inertgases wird über die Leitung (9) zwischen der Niedrigtemperatur- und der Hochtemperaturzone das bei der Reaktion entstandene Wasser zusammen mit etwaigen Zwischenprodukten (z. B. Monoacetylethylendiamin), vorerst ausgestrippt und vorzugsweise einer Fraktionierkolonne (5) zugeführt, wo das Reaktionswasser fraktioniert destilliert wird. Das in dieser Fraktionierkolonne anfallende Wasser wird über die Leitung (10) ausgetragen und der Rückstand über die Leitung (11) in die Hochtemperaturzone zurückgeführt. Das Inertgas entweicht am Kopf der Fraktionierkolonne über die Leitung (12). Die Verweilzeit des Reaktionsgemisches in dieser Apparatur beträgt ungefähr 3 bis 15, vorzugsweise 5 bis 6 Minuten.

Der Vorteil des erfindungsgemäßen Verfahrens ist darin zu sehen, daß hier im Vergleich zu dem in der DE-OS 2 118 282 beschriebenen Verfahren in einem stufenlos kontinuierlichen Prozeß kein Überschuß einer Reaktionskomponente erforderlich — wenngleich erlaubt — ist, somit Essigsäure und Ethylendiamin im stöchiometrischen Verhältnis eingesetzt werden können, und die Verweilzeit des Produktes gegenüber der beschriebenen Kaskadenschaltung auf wenige Minuten reduziert werden kann. Auf diese Weise ist es gelungen, den in der Behälterkaskade geschwindigkeitsbestimmenden Schritt — nämlich die destillative Entfernung des Reaktionswassers — so umzugestalten, daß die bisher vom Destillationsverhalten bestimmte Umsetzungsgeschwindigkeit auf die eigentliche Reaktionsgeschwindigkeit reduziert wird. Das entstandene Reaktionswasser wird im statu nascendi durch die große, während der Reaktion bereitgestellte Fläche verdampft und unmittelbar mit Hilfe des Inertgasstromes der Reaktionszone entzogen. Die Reaktionswasserentfernung ist somit nicht mehr bestimmend für die Reaktionsgeschwindigkeit wie das beim beschriebenen Kaskaden-Verfahren der Fall ist, so daß die Raum-Zeit-Ausbeute um etwa das 5- bis 10fache erhöht wird.

### Beispiel

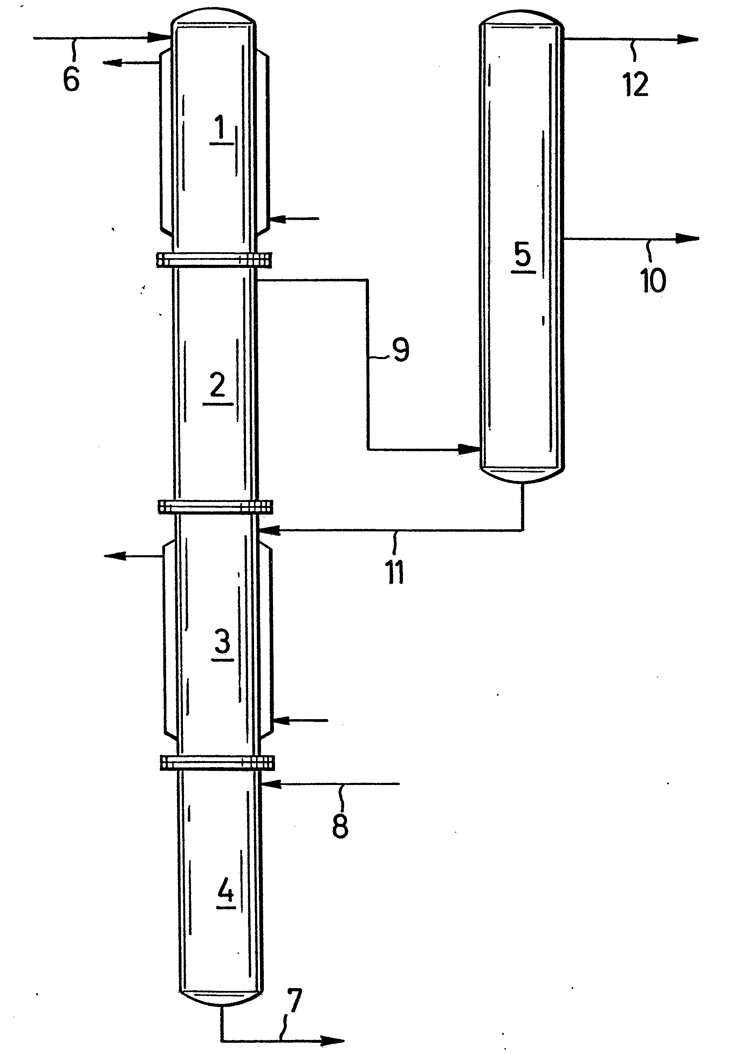

In eine Apparatur, wie sie in der Zeichnung schematisch dargestellt ist, wird pro Stunde ein Gemisch von 42 kg Essigsäure und 21 kg Ethylendiamin in die erste Füllkörperkolonne (1) eingegeben, die auf einer Temperatur von 90° C gehalten wird. Das Reaktionsgemisch läuft dann in die auf 180° C aufgeheizte Hochtemperaturzone, die aus einer ersten Heizzone (2), einer zweiten Füllkörperkolonne (3) und einer zweiten Heizzone (4) besteht. Beide Füllkörperkolonnen haben eine Länge von 1000 mm und eine Weite von 150 mm. Die Heizfläche in der ersten Heizzone beträgt 0,2 m² und in der zweiten Heizzone 0,4 m². Zwischen der Füllkörperkolonne (3) und der Heizzone (4) wird über die Leitung (8) kontinuierlich Stickstoff als Strippgas eingegeben (ca. 50 l/h) und das entstandene Reaktionswasser über die Leitung (9) ausgestrippt. In einer Fraktionierkolonne (5) wird das Reaktionswasser fraktioniert destilliert und der Rückstand wird über die Leitung (11) wieder in die Hochtemperaturzone zurückgeführt. Am Ende der zweiten Heizzone erhält man hierbei stündlich 50 kg reines Di-acetylethylendiamin.

### Patentanspruch

Verfahren zur kontinuierlichen Herstellung von Di-acetylethylendiamin, dadurch gekennzeichnet, daß man ein Gemisch aus Ethylendiamin und Essigsäure zunächst in einer Niedrigtemperaturzone bei ca. 80 bis 140° C vorreagieren läßt, das Reaktionsgemisch in einer nachfolgenden Hochtemperaturzone bei ca. 140 bis 215° C ausreagieren läßt, das gebildete Di-acetylethylendiamin am Ende der Hochtemperaturzone austrägt, das Reaktionswasser im Gegenstrom mit einem Inertgas zwischen der Niedrigtemperatur- und der Hochtemperaturzone ausstrippt und vorzugsweise das Reaktionswasser fraktioniert destilliert und die ausfraktionierten Reaktionskomponenten wieder in die Hochtemperaturzone zurückführt.

### Claim

A process for the continuous preparation of diacetyl ethylene diamine, which comprises allowing a mixture of ethylene diamine and acetic acid to react in a low-temperature zone at a temperature of about 80 to 140° C, completing the reaction of the mixture in a subsequent high-temperature zone of from about 140 to 215° C, discharging the diacetyl ethylene diamine obtained at the end of the high-temperature zone, stripping the reaction water in countercurrent flow by an inert gas at a point located between the low-temperature and the high-temperature zone and preferably subjecting the reaction water to fractional distillation and recycling the reaction components separated by fractionation to the high-temperature zone.

### Revendication

Procédé pour préparer en continu la diacétyléthylène-diamine, procédé caractérisé en ce qu'on fait d'abord subir un début de réaction (pré-réaction) à un mélange d'éthylène-diamine et d'acide acétique dans une zone où règne une température assez basse, en l'espèce une température comprise entre environ 80 et 140° C, puis on laisse la réaction s'achever dans une zone qui fait suite à la précédente et où règne une température plus élvée, en l'espèce une température comprise entre environ 140 et 215° C, on soutire en continu, à l'extrémité de la zone de haute température, la diacétyl-éthylène-diamine formée, on entraîne l'eau réactionnelle en faisant passer un gaz inerte à contrecourant entre la zone de basse température et la zone de haute température et on soumet de préférence l'eau réactionnelle à une distillation fractionnée, et on renvoit à la zone de haute température les composantes réactionnelles qui ont été séparées par fractionnement.